(19) Europäisches Patentamt
European Patent Office
Office européen des brevets

(11) Veröffentlichungsnummer : **0 077 541**
**B1**

# (12) EUROPÄISCHE PATENTSCHRIFT

(45) Veröffentlichungstag der Patentschrift : **05.06.85**

(51) Int. Cl.[4] : **C 07 J 71/00**, A 61 K 31/58

(21) Anmeldenummer : **82109551.0**

(22) Anmeldetag : **15.10.82**

(54) **Delta-1,3,5-3-Chlor-11-Beta,16-Alpha,17-Alpha,21-tetra-(hydroxy)-pregnan-20-on-16,17-acetonidderivate, Verfahren zu ihrer Herstellung und diese Verbindungen enthaltende Arzneimittel.**

(30) Priorität : **15.10.81 HU 297681**

(43) Veröffentlichungstag der Anmeldung : **27.04.83 Patentblatt 83/17**

(45) Bekanntmachung des Hinweises auf die Patenterteilung : **05.06.85 Patentblatt 85/23**

(84) Benannte Vertragsstaaten : **AT BE CH DE FR GB IT LI NL SE**

(56) Entgegenhaltungen :
**US-A- 2 985 652**
**US-A- 2 990 401**
**Die Akte enthält technische Angaben, die nach dem Eingang der Anmeldung eingereicht wurden und die nicht in dieser Patentschrift enthalten sind.**

(73) Patentinhaber : **Richter Gedeon Vegyészeti Gyár R.T.**
**Gyömröi ut 19-21**
**H-1475 Budapest X (HU)**

(72) Erfinder : **Tòth, Jòzsef, Dr.**
**Alsò-Törökvész ùt 10**
**Budapest II (HU)**
Erfinder : **Hajòs, György, Dr.**
**Gábor A. u. 59**
**Budapest II (HU)**
Erfinder : **Fekete, György, Dr.**
**Széher ùt 62**
**Budapest II (HU)**
Erfinder : **Szporny, Lászlò, Dr.**
**Szabolcska M. u. 7**
**Budapest XI (HU)**
Erfinder : **Horváth, István, Dr., Dipl.-chem.**
**Naphegy u. 33**
**Budapest I (HU)**
Erfinder : **Boòr, geb. Mezei, Anna, Dipl.-chem.**
**Mátyás kir. ùt 4**
**Budapest XII (HU)**
Erfinder : **Molnár, Csaba, Dipl.-chem.**
**Havanna u. 54**
**Budapest XVIII (HU)**
Erfinder : **Arányi, Péter, Dr., Dipl.-chem.**
**Nyéki ùt 9**
**Budapest II (HU)**
Erfinder : **Náray, Anikò, Dr.**
**Batthyány u. 3**
**Budapest I. (HU)**
Erfinder : **Görög, Sándor, Dr., Dipl.-chem.**
**Vajda Péter u. 43**
**Budapest VIII (HU)**

**EP 0 077 541 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Erfinder : **Holly, Sándor, Dr., Dipl.-chem.**
**Városhaz u. 14**
**Budapest V (HU)**
(74) Vertreter : **Beszédes, Stephan G. Dr.**
**Münchener Strasser 80a Postfach 1168**
**D-8060 Dachau (DE)**

**Beschreibung**

Die Erfindung betrifft neue $\Delta^{1,3,5}$-3-Chlor-11β,16α,17α,21-tetra-(hydroxy)-pregnan-20-on-16,17-acetonidderivate, ein Verfahren zu ihrer Herstellung und diese Verbindungen enthaltende Arzneimittel, insbesondere solche mit entzündungshemmender Wirkung.

Die Iminiumsalze werden in den letzten Jahren in der präparativen organischen Chemie in immer stärkerem Maße verwendet, wie dies zum Beispiel von H. Böhme und H. G. Viehe in ihrer Veröffentlichung « Iminium Salts in Organic Chemistry » (Advances in Organic Chemistry : Methodes and Results, Ed. : E. C. Taylor, Vol. 9, Part 1, J. Wiley and Sons Inc., 1976) im Kapitel « The Vilsmeier-Haack-Arnold Acylations » (Seiten 225 bis 333) ausführlich dargelegt ist.

Auch diese Veröffentlichung beweist überzeugend, daß die als « Vilsmeier-Reagenzien » bekannten Chlormethyleniminiumsalze der allgemeinen Formel

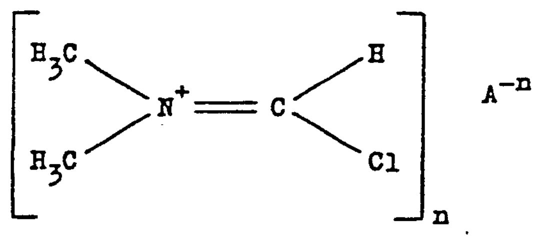

(III)

worin

$A^-$ für ein salzbildendes Anion, zum Beispiel Chlorid-, Bromid-, Dichlorphosphat-, Sulfat- oder Fluoboratanion steht und

n 1, 2 oder 3 ist,

zum Aufbau neuer Bindungen C—C durch Einführen einer Formylgruppe an ein reaktionsfähiges Kohlenstoffatom auf verschiedenste Weise genutzt werden können. Die Umsetzung fand auch Eingang in das Gebiet der Steroidchemie. Zum Beispiel können die 3-Enoläther der $\Delta^4$-3-(Oxo)-pregnanderivate in der 6-Stellung mit Vilsmeier-Reagenzien in guter Ausbeute formyliert werden. Je nachdem, welches Anion das Reagens aufweist, verläuft die Reaktion verschieden (Tetrahedron *25* [1969], 1 155). Die freien $\Delta^4$-3-(Oxo)-östranderivate sowie die $\Delta^4$-3-(Oxo)-androstan-, $\Delta^{4,6}$-3-(Oxo)-östran- beziehungsweise $\Delta^{4,6}$-3-(Oxo)-androstanderivate liefern bei dieser Umsetzung unterschiedlich formylierte 3-(Chlor)-steroiddiene beziehungsweise -triene (Tetrahedron Lett. *1965*, 137 ; Chem. Ber. *101* [1968], 2 393). Aus den 5α-Androstan-3-onderivaten werden unter energischen Reaktionsbedingungen die entsprechenden 3-(Chlor)-2,4-di-(formyl)-derivate erhalten (J. Chem. Soc. *1965*, 788), während die 19-Nor-pregna-4,6-dien-3,20-dionderivate nicht nur chloriert und formyliert werden, sondern in ihrem A-Ring auch noch eine Aromatisierung erfahren (Chem. Ber. *101* [1968], 2 393).

Ferner sind aus der US-A-2 985 652 3-(Alkoxy)-6α,9α-di-(fluor)-11β,16β,17β,21-tetra-(hydroxy)-pregna-3,5-dien-20-on-16,17-acetonide und 3-(Alkoxy)-6α-(fluor)-11β,16α,17α,21-tetra-(hydroxy)-pregna-3,5-dien-20-on-16,17-acetonide und ihre 21-Acetate sowie ihre entzündungshemmende Wirkung ohne nähere Angaben bekannt. Diese haben in der 3-Stellung zwingend einen Alkoxyrest. Sie können zwischen den 1- und 2-Stellungen keine Doppelbindung haben.

Weiterhin sind in der US-A-2 990 401 unter anderen 9α-(Fluor)-11β,16α,17α,21-tetra-(hydroxy)-pregna-1,4-dien-3,20-dion-16,17-acetonide, wie 9α-Fluor-11β,16α,17α,21-tetra-(hydroxy)-pregna-1,4-dien-3,20-dion-16,17-acetonid und ihre 21-Alkansäureester sowie ihre entzündungshemmende Wirkung ohne nähere Angaben beschrieben.

Der Erfindung liegt die Aufgabe zugrunde, überlegene pharmakologische Wirkungen aufweisende neue 3-(Chlor)-pregnanderivate, ein Verfahren zur Herstellung derselben und diese Verbindungen enthaltende Arzneimittel zu schaffen.

Das Obige wurde überraschenderweise durch die Erfindung erreicht.

Gegenstand der Erfindung sind $\Delta^{1,3,5}$-3-Chlor-11β,16α,17α,21-tetra-(hydroxy)-pregnan-20-on-16,17-acetonidderivate der allgemeinen Formel

(Siehe Figur Seite 4 f.)

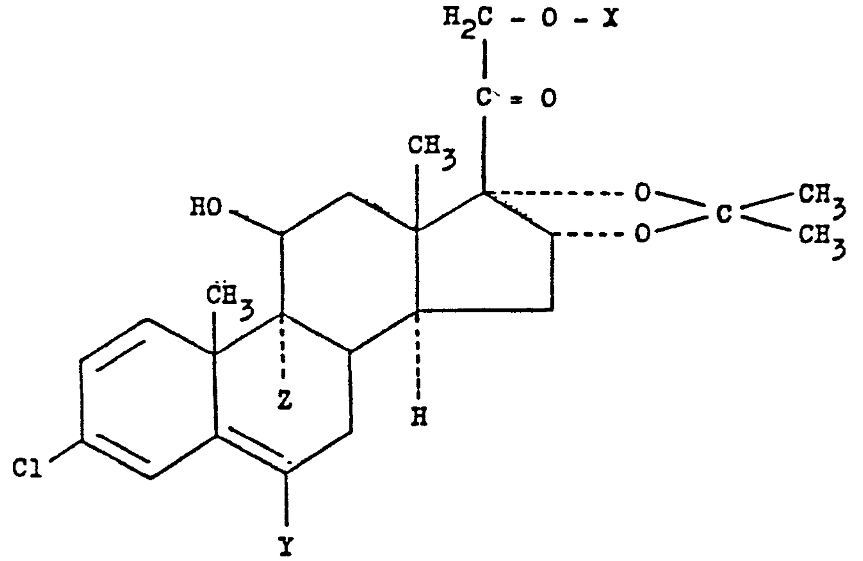

(I)

worin

X für ein Wasserstoffatom oder einen Acetyl- oder Monochloracetylrest steht und

Y und Z jeweils ein Halogen- beziehungsweise Wasserstoffatom bedeuten,

mit der weiteren Maßgabe, daß mindestens 1 von Y und Z von Wasserstoff verschieden ist.

Vorzugsweise ist beziehungsweise sind das beziehungsweise die Halogenatom(e), für welches beziehungsweise welche Y und/oder Z stehen kann beziehungsweise können, [ein] Fluor-, Brom- und/oder Chloratom(e), wobei die ersten beiden besonders bevorzugt sind.

Besonders bevorzugt sind diejenigen erfindungsgemäßen $\Delta^{1,3,5}$-3-Chlor-11β,16α,17α,21-tetra-(hydroxy)-pregnan-20-on-16,17-acetonidderivate der allgemeinen Formel I, bei welchen Y für ein Wasserstoff-, Fluor- oder Bromatom steht und Z ein Fluoratom bedeutet.

Ganz besonders bevorzugte erfindungsgemäße Verbindungen sind 3-(Chlor)-9α-(fluor)-11β,16α,17α,21-tetra-(hydroxy)-pregna-1,3,5-trien-20-on-16,17-acetonid, 3-(Chlor)-6,9α-di-(fluor)-11β,16α,17α,21-tetra-(hydroxy)-pregna-1,3,5-trien-20-on-16,17-acetonid und 3-(Chlor)-6-(brom)-9α-(fluor)-11β,16α,17α,21-tetra-(hydroxy)-pregna-1,3,5-trien-20-on-16,17-acetonid.

Ferner wurde im Zuge der Forschungen, welche auf die Herstellung dieser vorteilhafte Eigenschaften aufweisenden neuen $\Delta^{1,3,5}$-3-Chlor-11β,16α,17α,21-tetra-(hydroxy)-pregnan-20-on-16,17-acetonidderivate gerichtet waren, das Verhalten von $\Delta^{1,4}$-3-(Oxo)-pregnanderivaten gegenüber Vilsmeier-Reagenzien untersucht. Es ist bekannt, daß die Reaktionsfähigkeit der von trans/anti/trans/anti/trans-raumorientiert aneinanderkondensierte Ringe aufweisenden beziehungsweise anellierten Pregnanderivaten ableitbaren $\Delta^{1,4}$-3-(Oxo)-derivate sehr gering ist. Diese Oxogruppe bildet keine Enoläther, Enolester, Enamine beziehungsweise offene oder ringförmige Ketale, obwohl doch die $\Delta^{4}$-3-(Oxo)-derivate diese Reaktionen im allgemeinen in guter Ausbeute eingehen (J. Fried und J. A. Edwards : « Organic Reactions in Steroid Chemistry », van Nostrand Reinhold Co. 1972, Seite 394).

Im Laufe dieser Forschungen wurde nun die überraschende Feststellung gemacht, daß beim Umsetzen von $\Delta^{1,4}$-11β,16α,17α,21-Tetra-(hydroxy)-pregnan-3,20-dion-16,17-acetonid-21-acetat- beziehungsweise $\Delta^{1,4}$-11β,16α,17α,21-Tetra-(hydroxy)-pregnan-3,20-dion-16,17-acetonid-21-monochloracetatderivaten der allgemeinen Formel

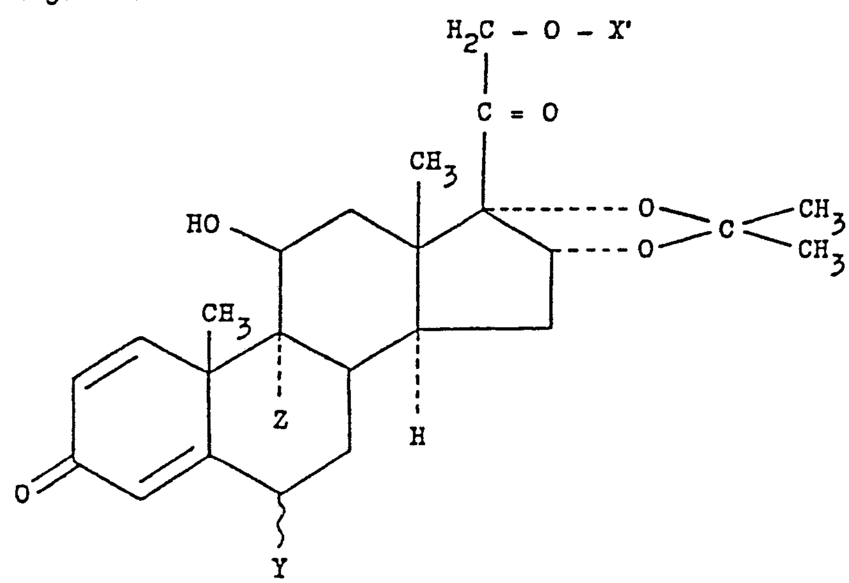

(II)

worin

X' für einen Acetyl- oder Monochloracetylrest steht und

Y und Z wie oben festgelegt sind,

mit Vilsmeier-Reagenzien die wenig reaktionsfähige 3-Oxogruppe der Pregnanderivate beseitigt wird, eine Bindung C3-Cl entsteht und sich ein aus 3 ungesättigten Bindungen bestehendes Doppelbindungssystem, dessen Doppelbindungen sich in den Ringen A und B des Steroidgerüstes befinden, bildet.

Ferner wurde ebenfalls überraschenderweise festgestellt, daß beim Umsetzen dieser $\Delta^{1,4}$-11β,16α,17α,21-Tetra-(hydroxy)-pregnan-3,20-dion-16,17-acetonid-21-acetat- beziehungsweise $\Delta^{1,4}$-11β,16α,17α,21-Tetra-(hydroxy)-pregnan-3,20-dion-16,17-acetonid-21-monochloracetatderivate nur Vilsmeier Reagenzien in aprotonischen organischen Lösungsmitteln in Gegenwart von tertiären Basen außer dem Austausch der Oxogruppe gegen das Chloratom und der Ausbildung der 3 Doppelbindungen keine weiteren Reaktionen im Molekül ablaufen. Diese Selektivität ist überraschend, denn aus dem oben erörterten Schrifttum (Tetrahedron Lett. *1965*, 137 ; Chem. Ber. *101* [1968], 2 393) ist bekannt, daß bei der Umsetzung von 3-(Oxo)-steroiden mit Vilsmeier-Reagenzien die ersteren außer dem Austausch der 3-Oxogruppe gegen Chlor in jedem Falle 1- oder mehrfach formyliert werden. Außerdem ist es auch überraschend, daß auch die Hydroxygruppe in der 11-Stellung der $\Delta^{1,4}$-11β,16α,17α,21-Tetra-(hydroxy)-pregnan-3,20-dion-16,17-acetonid-21-acetat- beziehungsweise $\Delta^{1,4}$-11β,16α,17α,21-Tetra-(hydroxy)-pregnan-3,20-dion-16,17-acetonid-21-monochloracetatderivate der allgemeinen Formel II unverändert bleibt, denn gemäß dem oben erörterten Schrifttum (Tetrahedron Letters *25* [1969], 1 155) wird bei der Umsetzung der dort abgehandelten $\Delta^4$-3-(Oxo)-pregnanderivate mit Vilsmeier-Reagenzien die Hydroxygruppe in der 11-Stellung derart schnell formyliert, daß die freien 11-Hydroxyderivate nur in sehr geringen Ausbeuten erhalten werden können.

Gegenstand der Erfindung ist daher auch ein Verfahren zur Herstellung der erfindungsgemäßen Verbindungen, bei welchem $\Delta^{1,4}$-11β,16α,17α,21-Tetra-(hydroxy)-pregnan-3,20-dion-16,17-acetonid-21-acetat- beziehungsweise $\Delta^{1,4}$-11β,16α,17α,21-Tetra-(hydroxy)-pregnan-3,20-dion-16,17-acetonid-21-monochloracetatderivate der allgemeinen Formel

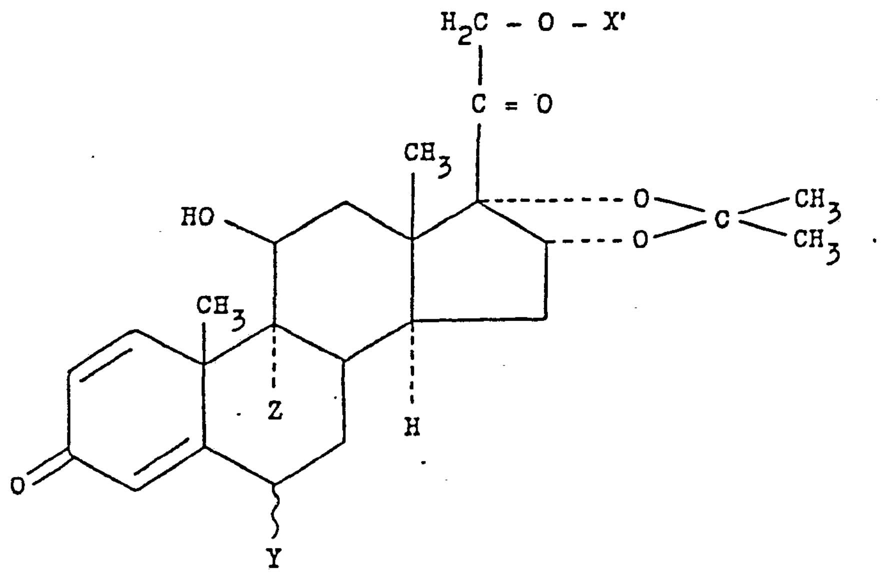

(II)

worin

X' für einen Acetyl- oder Monochloracetylrest steht und

Y und Z wie oben festgelegt sind,

in aprotonischen (aprotischen) Lösungsmitteln in Gegenwart von 1 oder mehr tertiären Base(n), insbesondere tertiären Amin(en), mit Chlormethyleniminiumsalzen der allgemeinen Formel

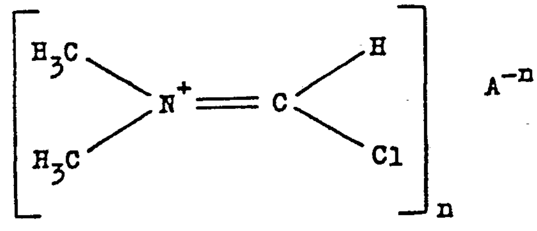

(III)

worin

A⁻ für ein salzbildendes Anion steht und

n 1, 2 oder 3, insbesondere 3, ist,

zu $\Delta^{1,3,5}$-3-Chlor-11β,16α,17α,21-tetra-(hydroxy)-pregnan-20-on-16,17-acetonidderivaten der allgemeinen Formel I, bei welchen X für einen Acetyl- oder Monochloracetylrest steht, umgesetzt werden und gegebenenfalls die letzteren in an sich bekannter Weise zu den entsprechenden $\Delta^{1,3,5}$-3-Chlor-11β,16α,17α,21-tetra-(hydroxy)-pregnan-20-on-16,17-acetonidderivaten der allgemeinen Formel I, bei welchen X für Wasserstoff steht, hydrolysiert werden.

Vorzugsweise werden als Chlormethyleniminiumsalze der allgemeinen Formel III solche, bei welchen $A^-$ für ein Dichlorphosphatanion

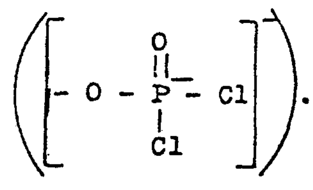

steht, verwendet.

Es ist auch bevorzugt, als aprotonische(s) Lösungsmittel 1 oder mehr niedere(n) Chlorkohlenwasserstoff(e) und/oder Dimethylformamid zu verwenden.

Ferner ist es bevorzugt, als tertiäre Base Pyridin und/oder 1 oder mehr Monoalkyl- und/oder Polyalkylderivat(e) desselben zu verwenden.

Zweckmäßig wird das erfindungsgemäße Verfahren wie folgt durchgeführt.

Das als Ausgangsverbindung eingesetzte Chlormethyleniminiumsalz der allgemeinen Formel III wird in einem wasserfreien organischen Lösungsmittel, vorteilhaft jeweils im gleichen, in welchem auch das betreffende Chlormethyleniminiumsalz der allgemeinen Formel III hergestellt worden ist, gelöst, zur Lösung wird beziehungsweise werden 1 oder mehr tertiäre Base(n), wie bereits erwähnt vorzugsweise Pyridin und/oder 1 oder mehr seiner Homologen, wie Picolin(e), Lutidin(e) und/oder Kollidin(e), zugegeben und das Gemisch wird bei Temperaturen von – 10 °C bis Raumtemperatur, insbesondere – 10 bis 0 °C, mit einer auf die untern beschriebene Weise bereiteten Lösung des Vilsmeier-Reagens versetzt. Das Vilsmeier-Reagens wird vorzugsweise im Überschuß eingesetzt ; insbesondere werden etwa 3 Mol Chlormethyleniminiumsalz der allgemeinen Formel III je Mol $\Delta^{1,3,5}$-3-Chlor-11β,16α,17α,21-tetra-(hydroxy)-pregnan-20-on-16,17-acetonidderivat der allgemeinen Formel II verwendet. Die Umsetzung dauert je nach den verwendeten Ausgangsstoffen, 20 bis 30 Minuten bis 5 Stunden. Die Temperatur des Reaktionsgemisches wird vom Anfangswert während der Reaktion auf Raumtemperatur ansteigen gelassen.

Nach dem Ende der Reaktion wird das Reaktionsgemisch durch Zugabe einer Base, zum Beispiel einer wäßrigen Kaliumbicarbonatlösung, zersetzt. Anschließend wird die organische Phase abgretennt und die wäßrige Phase mit einem mit Wasser nicht mischbaren Lösungsmittel extrahiert. Die vereinigten organischen Phasen werden bis zur Erreichung der neutralen Reaktion gewaschen und getrocknet und das Lösungsmittel wird abgedampft. Das erhaltene $\Delta^{1,3,5}$-3-Chlor-11β,16α,17α,21-tetra-(hydroxy)-pregnan-20-on-16,17-acetonidderivat der allgemeinen Formel I, bei welchem X für einen Acetyl- oder Monochloracetylrest steht, wird isoliert.

Die gegebenenfalls erfolgende Hydrolyse des erhaltenen $\Delta^{1,3,5}$-3-Chlor-11β,16α,17α,21-tetra-(hydroxy)-pregnan-20-on-16,17-acetonidderivates der allgemeinen Formel I, bei welchem X für einen Acetyl- oder Chloracetylrest steht, zum entsprechenden $\Delta^{1,3,5}$-3-Chlor-11β,16α,17α,21-tetra-(hydroxy)-pregnan-20-on-16,17-acetonidderivat, bei welchem X Wasserstoff bedeutet, kann als saure oder basische Hydrolyse durchgeführt werden. Vorzugsweise wird in einem mit Wasser zumindest teilweise mischbaren Lösungsmittel, zum Beispiel Alkohol, oder in einem zum Lösen von Steroiden geeigneten Lösungsmittel, zum Beispiel einem Gemisch aus Benzol und einem Alkohol, bei einer Temperatur von Raumtemperatur bis zum Siedepunkt des Reaktionsgemisches hydrolysiert. Im Falle der basischen Hydrolyse wird als Base vorteilhaft ein Alkalicarbonat oder -bicarbonat eingesetzt, während zur Vornahme der sauren Hydrolyse zum Beispiel Mineralsäuren, wie Salzäure, Schwefelsäure oder Überchlorsäure, oder organische Säuren, zum Beispiel Ameisensäure, Essigsäure oer Trifluoressigsäure, verwendet werden können.

Im Falle der Herstellung von $\Delta^{1,3,5}$-3-Chlor-11β,16α,17α,21-tetra-(hydroxy)-pregnan-20-on-16,17-acetonid-derivaten der allgemeinen Formel I, bei welchen X Wasserstoff bedeutet, wird in der ersten Stufe des erfindungsgemäß Verfahrens, das heißt bei der Umsetzung der $\Delta^{1,4}$-11β,16α,17α,21-Tetra-(hydroxy)-pregnan-3,20-dion-16,17-acetonid-21-acetat- beziehungsweise $\Delta^{1,4}$-11β,16α,17α,21-Tetra-(hydroxy)-pregnan-3,20-dion-16,17-acetonid-21-monochloracetatderivate der allgemeinen Formel II mit den Vilsmeier-Reagenzien Chlormethyleniminiumsalze der allgemeinen Formel III, die Acylgruppe zum Schutz der Hydroxygruppe in der 21-Stellung zweckmäßig so gewählt, daß ihr hydrolytisches Abspalten unter Bedingungen, welche die Substituenten, für welche Y und Z stehen, nicht angreifen, vorgenommen werden kann. So wird zur Herstellung der $\Delta^{1,3,5}$-3-Chlor-11β,16α,17α,21-tetra-(hydroxy)-pregnan-20-on-16,17-acetonidderivate der allgemeinen Formel I, bei welchen sowohl Y als auch Z je 1 Fluoratom bedeuten und X für Wasserstoff steht, vorteilhaft von den entsprechenden $\Delta^{1,4}$-11β,16α,17α,21-Tetra-(hydroxy)-pregnan-3,20-dion-16,17-acetonid-21-acetat-beziehungsweise $\Delta^{1,4}$-11β,16α,17α,21-Tetra-(hydroxy)-pregnan-3,20-dion-16,17-acetonid-21-monochloracetatderivate der allgemeinen Formel II, bei welchen Y und Z für Fluoratome stehen und X' einen Monochloracetylrest bedeutet, ausgegangen und die Hydrolyse in einem schwach alkalischen Medium durchgeführt. Zum Beispiel kann das erhaltene 3-(Chlor)-21-(monochloracetoxy)-derivat 3-(Chlor)-6,9α-di-(fluor)-11β,16α,17α,21-tetra-(hydroxy)-pregna-1,3,5-trien-20-on-16,17-acetonid-21-(monochloracetat) in einem Gemisch aus Methanol und Benzol gelöst und mit einer Lösung eines Alkalicarbonates oder Alkalibicarbonates hydrolysiert werden. Zur Herstellung der $\Delta^{1,3,5}$-3-Chlor-11β,16α,17α,21-tetra-(hydroxy)-pregnan-20-on-16,17-acetonidderivate der allgemeinen Formel I, bei welchen Y für Brom steht, Z Fluor bedeutet und X Wasserstoff darstellt, ist es dagegen am vorteilhaftesten, von den entsprechenden $\Delta^{1,4}$-11β,16α,17α,21-Tetra-(hydroxy)-pregnan-3,20-dion-16,17-acetonid-21-acetat- beziehungsweise $\Delta^{1,4}$-11β,16α,17α,21-Tetra-(hydroxy)-pregnan-3,20-dion-16,17-acetonid-21-monochloracetatderivaten der allgemeinen Formel II, bei welchen Y für Brom steht, Z Fluor bedeutet und X' einen Acetylrest darstellt, auszugehen. Das in der ersten Reaktionsstufe erhaltene 3-(Chlor)-21-(acetoxy)-steroid 3-(Chlor)-6-(brom)-9α-(fluor)-11β,16α,17α,21-tetra-(hydroxy)-pregna-1,3,5-trien-20-on-16,17-acetonid-21-(acetat) kann dann zum Beispiel in Methanol gelöst oder suspendiert und durch Zugabe einer wäßrigen Säure hydrolysiert werden.

Das nach dem Ende der Hydrolyse erhaltene Reaktionsgemisch kann in an sich bekannter Weise, zum Beispiel durch Extrahieren des erhaltenen Produktes mit einem mit Wasser nicht mischbaren Lösungsmittel, Waschen bis zum Erreichen der neutralen Reaktion, Trocknen und Eindampfen des Lösungsmittels, aufgearbeitet werden.

Die im erfindungsgemäßen Verfahren als Ausgangsstoffe verwendeten $\Delta^{1,4}$-11β,16α,17α,21-Tetra-(hydroxy)-pregnan-3,20-dion-16,17-acetonid-21-acetat- beziehungsweise $\Delta^{1,4}$-11β,16α,17α,21-Tetra-(hydroxy)-pregnan-3,20-dion-16,17-acetonid-21-monochloracetatderivate der algemeinen Formel II können in der Weise hergestellt worden sein, daß die entsprechenden $\Delta^{1,4}$-11β,16α,17α,21-Tetra-(hydroxy)-pregnan-3,20-dion-16,17-acetonidderivate der in der Weise abgewandelten allgemeinen Formel II, daß an Stelle von X' Wasserstoff ist, welche im allgemeinen bekannte Verbindungen sind, in an sich bekannter Weise an der Hydroxygruppe in der 21-Stellung selektive acyliert werden. Die zweiten Ausgangsmaterialien des erfindungsgemäßen Verfahrens, die Vilsmeier-Reagenzien Chlormethyleniminiumsalze der allgemeinen Formel III, können zweckmäßig durch Umsetzen von Dimethylformamid und Phosphoroxychlorid in wasserfreien aprotonischen Lösungsmitteln in situ hergestellt worden sein. Als aprotonische Lösungsmittel werden vorteilhaft niedere Halogenkohlenwasserstoffe, insbesondere Dichlormethan und/oder Chloroform, verwendet.

Ferner sind erfindungsgemäß Arzneimittel, welche 1 oder mehr der erfindungsgemäßen Verbindung(en) Wirkstoff beziehungsweise Wirkstoffe, zweckmäßig zusammen mit 1 oder mehr üblichen pharmazeutischen Konfektionierungsmittel(n), enthalten, vorgesehen.

Die erfindungsgemäßen $\Delta^{1,3,5}$-3-Chlor-11β,16α,17α,21-tetra-(hydroxy)-pregnan-20-on-16,17-acetonidderivate der allgemeinen Formel I haben nämlich wie bereits erwähnt eine wertvolle entzündungshemmende Wirkung, und zwar vom Glucocorticoidtyp. In den im folgenden beschriebenen Versuchen wurden die erfindungsgemäßen Verbindungen 3-(Chlor)-9α-(fluor)-11β,16α,17α,21-tetra-(hydroxy)-pregna-1,3,5-trien-20-on-16,17-acetonid [3-Chlor-TCA] {Beispiel 2 oder 4}, 3-(Chlor)-6,9α-di-(fluor)-11β,16α,17α,21-tetra-(hydroxy)-pregna-1,3,5-trien-20-on-16,17-acetonid [3-Chlor-FCA] {Beispiel 6} und 3-(Chlor)-6-(brom)-9α-(fluor)-11β,16α,17α,21-tetra-(hydroxy)-pregna-1,3,5-trien-20-on-16,17-acetonid [3-Chlor-6-brom-TCA] {Beispiel 7} und als Vergleichssubstanzen die bekannten Verbindungen 9α-(Fluor)-11β,16α,17α,21-tetra-(hydroxy)-pregna-1,4-dien-3,20-dion-16,17-acetonid [TCA] {Vergleichssubstanz A} und 6α,9α-Di-(fluor)-11β,16α,17α,21-tetra-(hydroxy)-pregna-1,4-dien-3,20-dion-16,17-acetonid [FCA] {Vergleichssubstanz B} gleicher Wirkungsrichtung untersucht.

Es wurde die Affinität der genannten Verbindungen zu den Glucocorticoid-Rezeptoren der Rattenleber (Baxter, J. D., Tomkins, G. M. : Specific cytoplasmic glucocorticoid receptors in hepatoma tissue culture cells, Proc. Natl. Acad. Sci. USA *68* [1971], 932 bis 937) untersucht, denn diese Affinität ist die Voraussetzung der glucocorticoiden Wirkung. Gemäß dem Schrifttum beträgt die scheinbare Dissoziationskonstante des 9α-(Fluor)-11β,16α,17α,21-tetra-(hydroxy)-pregna-1,4-dien-3,20-dion-16,17-acetonides [TCA] {Vergleichssubstanz A} 10 nM (A. Munck und K. Leung : Receptors and Mechanism of Action of Steroid Hormones ; Ed. J. R. Pasqualini ; Marcell Decker, New York, 1977, Seite 343). Die scheinbare Dissoziationskonstante des 3-(Chlor)-9α-(fluor)-11β,16α,17α,21-tetra-(hydroxy)-pregna-1,3,5-trien-20-on-16,17-acetonides [3-Chlor-TCA] {Beispiel 2 oder 4} ist 30 nM, die des 6α,9α,Di-(fluor)-11β,16α,17α,21-tetra-(hydroxy)-pregna-1,4-dien-3,20-dion-16,17-acetonides ⟨Fluocinolon-acetonides⟩[FCA] {Vergleichssubstanz B} 15 nM und die des 3-(Chlor)-6,9α-di-(fluor)-11β,16α,17α,21-tetra-(hydroxy)-pregna-1,3,5-trien-20-on-16,17-acetonides [3-Chlor-FCA] {Beispiel 6} 25 nM, das heißt ein ähnlicher Wert. Die scheinbare Dissoziationskonstante des 3-(Chlor)-6-(brom)-9α-(fluor)-11β,16α,17α,21-tetra-(hydroxy)-pregna-1,3,5-trien-20-on-16,17-acetonides [3-Chlor-6-brom-TCA] {Beispiel 7} beträgt 60 nM.

Zur Erhärtung der Ergebnisse der mit Rezeptorbindung vorgenommenen Versuche wurde an Küken und an Ratten auch die Wirkung von 9α-(Fluor)-11β,16α,17α,21-tetra-(hydroxy)-pregna-1,4-dien-3,20-dion-16,17-acetonid [TCA] {Vergleichssubstanz A} US-A-2 990 401-Beispiel 12 und 3-(Chlor)-9α-(fluor)-11β,16α,17α,21-tetra-(hydroxy)-pregna-1,3,5-trien-20-on-16,17-acetonid [3-Chlor-TCA] {Beispiel 2 oder 4} auf die Induktion der Tyrosinaminotransferase untersucht (Diamandstone, T. I. : Assay of tyrozine transaminase activity by conversion of p-Hydroxyphenylpyruvate to Hydroxybenzaldehyde, Anal. Biochem. *16* [1966], 395 bis 401). 9α-(Fluor)-11β,16α,17α,21-tetra-(hydroxy)-pregna-1,4-dien-3,20-dion-16,17-acetonid [TCA] {Vergleichssubstanz A} US-A-2 990 401-Beispiel 12 zeigte in Dosen von 0,15 µg/mg beziehungsweise 0,025 µg/mg eine 50 %-ige induzierende Wirkung. 3-(Chlor)-9α-(fluor)-11β,16α,17α,21-tetra-(hydroxy)-pregna-1,3,5-trien-20-on-16,17-acetonid [3-Chlor TCA] {Beispiel 2 oder 4} zeigte in den kleineren Dosen von 0,05 beziehungsweise 0,005 mg/100 g eine ähnliche Wirkung.

Es ist bekannt, daß die Glucocorticoide eine Tymusrückbildung beziehungsweise -involution herbeiführen (Greengard, O., Machovich, R. : Hydrocortisone regulation of thymidine kinase in thymus involution and hematopoietic tissues, Biochem. Biophys. Acta *286* [1972], 382 bis 388). Ein 50 %-iger Rückgang des Thymusgewichtes konnte sowohl mit 9α-(Fluor)-11β,16α,17α,21-tetra-(hydroxy)-pregna-1,4-dien-3,20-dion-16,17-acetonid [TCA] {Vergleichssubstanz A} als auch mit 3-(Chlor)-9-(fluor)-11β,16α,17α,21-tetra-(hydroxy)-pregna-1,3,5-trien-20-on-16,17-acetonid [3-Chlor-TCA] {Beispiel 2 oder 4} bei Küken mit einer Dosis von 0,01 mg/100 g und bei Ratten mit einer Dosis von 0,001 mg/100 g erreicht werden.

Weitere Untersuchungen :

I) Systemische entzündungshemmende Wirkung

a) Hemmung des Carraghenin-Sohlenödemes (Winter und Mitarbeiter : J. Pharm. Exp. Therap. *141* [1963], 369)

Tabelle 1

| Substanz Beispiel Nr. | Bezeichnung | Perorale Dosis in mg/kg | Hemmung in % |
|---|---|---|---|
| 1 | 3-(Chlor)-9α-(fluor)-11ß,16α,17α,21-tetra-(hydroxy)-pregna-1,3,5-trien-20-on-16,17-acetonid-21-acetat [3-Chlor-TCA-21-acetat] | 2 | 8,9 |
| | | 6 | 9,6 |
| 2 oder 4 | 3-(Chlor)-9α-(fluor)-11ß,16α,17α,21-tetra-(hydroxy)-pregna-1,3,5-trien-20-on-16,17-acetonid [3-Chlor-TCA] | 2 | 30,2 |
| | | 6 | 38,7 |

b) Verfahrensweise mit dem Granulomsack nach Selye (Selye, J. : Resent Progr. Horm. Res. *8* [1953], 117)

Tabelle 2

| Substanz Beispiel Nr. | Bezeichnung | Subkutane Dosis in mg/kg | Hemmung in % |
|---|---|---|---|
| 1 | 3-(Chlor)-9α-(fluor)-11ß,16α,17α,21-tetra-(hydroxy)-pregna-1,3,5-trien-20-on-16,17-acetonid-21-acetat [3-Chlor-TCA-21-acetat] | 0,5 | 56,6 |
| | | 1,5 | 73,3 |

Tabelle 2 (Fortsetzung)

| Substanz Beispiel Nr. | Bezeichnung | Subkutane Dosis in mg/kg | Hemmung in % |
|---|---|---|---|
| 2 oder 4 | 3-(Chlor)-9α-(fluor)-11ß,16α,17α,21-tetra-(hydroxy)-pregna-1,3,5-trien-20-on-16,17-acetonid [3-Chlor-TCA] | 0,5 | 90,8 |
| | | 1,5 | 90,1 |

c) Verfahrensweise mit dem Wattegranulom (Winter, C. A. und Mitarbeiter : J. Pharm. Exp. Therap. *141* [1963], 369)

Tabelle 3

| Substanz Beispiel Nr. | Bezeichnung | Subkutane Dosis in mg/kg | Hemmung in % |
|---|---|---|---|
| 1 | 3-(Chlor)-9α-(fluor)-11ß,16α,17α,21-tetra-(hydroxy)-pregna-1,3,5-trien-20-on-16,17-acetonid-21-acetat [3-Chlor-TCA-21-acetat] | 0,5 | 33,0 |
| | | 1,5 | 91,0 |
| 2 oder 4 | 3-(Chlor)-9α-(fluor)-11ß,16α,17α,21-tetra-(hydroxy)-pregna-1,3,5-trien-20-on-16,17-acetonid [3-Chlor-TCA] | 0,5 | 22,0 |
| | | 1,5 | 26,0 |

II) Örtliche entzündungshemmende Wirkung

a) Sackverfahrensweise nach Selye für lokale Untersuchungen (Bianchetti und Mitarbeiter : Arzneim. Forsch. *27* [1977], 2 096)

Tabelle 4

| Substanz Beispiel Nr. | Bezeichnung | Dosis in mg/Sack | Hemmung in % |
|---|---|---|---|
| 1 | 3-(Chlor)-9α-(fluor)-11ß,16α,17α,21-tetra-(hydroxy)-pregna-1,3,5-trien-20-on-16,17-acetonid-21-acetat [3-Chlor-TCA-21-acetat] | 0,033 | 12,4 |
| | | 0,10 | 26,3 |
| 2 oder 4 | 3-(Chlor)-9α-(fluor)-11ß,16α,17α,21-tetra-(hydroxy)-pregna-1,3,5-trien-20-on-16,17-acetonid [3-Chlor-TCA] | 0,033 | 26,0 |
| | | 0,10 | 32,3 |

b) Verfahrensweise des örtlichen Wattegranulomes (Silvestrini, B. : Arzneim. Forsch. *19* [1969], 30)

Tabelle 5

| Substanz Beispiel Nr. | Bezeichnung | Dosis in mg/Watte | Hemmung in % |
|---|---|---|---|
| 1 | 3-(Chlor)-9α-(fluor)-11ß,16α,17α,21-tetra-(hydroxy)-pregna-1,3,5-trien-20-on-16,17-acetonid-21-acetat [3-Chlor-TCA-21-acetat] | 0,033 | 52,4 |
| | | 0,10 | 35,3 |
| 2 oder 4 | 3-(Chlor)-9α-(fluor)-11ß,16α,17α,21-tetra-(hydroxy)-pregna-1,3,5-trien-20-on-16,17-acetonid [3-Chlor-TCA] | 0,033 | 24,1 |
| | | 0,10 | 51,4 |

c) Hemmung der durch Crotonöl ausgelösten Ohrenentzündung (Tonelli und Mitarbeiter : Endocrinology *77* [1965] 625)

Tabelle 6

| Substanz Beispiel Nr. | Bezeichnung | Dosis in $mg/cm^3$ | Hemmung in % |
|---|---|---|---|
| 1 | 3-(Chlor)-9α-(fluor)-11ß,16α,17α,21-tetra-(hydroxy)-pregna-1,3,5-trien-20-on-16,17-acetonid-21-acetat [3-Chlor-TCA-21-acetat] | 0,02 | 10,0 |
| | | 0,10 | 17,8 |
| 2 oder 4 | 3-(Chlor)-9α-(fluor)-11ß,16α,17α,21-tetra-(hydroxy)-pregna-1,3,5-trien-20-on-16,17-acetonid [3-Chlor-TCA] | 0,02 | 30,0 |
| | | 0,10 | 52,9 |

Die Erfindung wird an Hand der folgenden Beispiele näher erläutert.

## Beispiel 1

3-Chlor-9α-fluor-11β,16α,17α,21-tetrahydroxy-pregna-1,3,5-trien-20-on-16,17-acetonid-21-acetat

In ein Gemisch aus 120 ml Dichlormethan und 45 ml Dimethylformamid werden bei − 10 °C 14 ml (152,0 mMol) Phosphoroxychlorid eingetropft. Das Gemisch wird bei − 10 °C 20 Minuten lang stehen gelassen und dann bei einer Temperatur unter 0 °C mit der Lösung von 23,8 g (48,5 mMol) 9α-Fluor-11β,16α,17α,21-tetrahydroxy-pregna-1,4-dien-3,20-dion-16,17-acetonid-21-acetat und 0,5 ml Pyridin in 200 ml Dichlormethan versetzt. Nach 20 minütigem Rühren wird die erhaltene Lösung mit weiteren 200 ml Dichlormethan verdünnt und dann in die mit 1 200 ml Eiswasser bereitete Lösung von 60,9 g Kaliumhydrogencarbonat eingegossen. Das Gemisch wird 30 Minuten lang gerührt, dann werden die Phasen voneinander getrennt, und die wäßrige Phase wird mit 2 × 200 ml Dichlormethan ausgeschüttelt. Die organischen Phasen werden vereinigt, mit Wasser gewaschen, über wasserfreiem Natriumsulfat getrocknet und dann eingedampft. Das als Rückstand erhaltene Rohprodukt wird in 200 ml Aceton gelöst und die Lösung in 2 Liter eiskalte wäßrige 10 %ige Natriumchloridlösung eingetropft. Nach dem Verrühren wird das ausgeschiedene Produkt abfiltriert, mit Wasser gewaschen und bei Raumtemperatur, vor Licht geschützt, über Phosphorpentoxyd im Vakuum getrocknet. 24,2 g (97,8 % der theoretischen Ausbeute) der Verbindung werden in Form einer gelben Substanz erhalten, die bei 187-190 °C unter Zersetzung schmilzt. Nach Umkristallisieren aus Aceton und anschließend aus Methanol schmilzt die Substanz unter Zersetzung bei 222-228 °C.

Elementaranalyse :

Cl gefunden : 6,65 %, 6,83 % (berechnet : 7,16 %)

IR : 3 460 (υ-OH), 1 755 (υ>C = O, Acetat), 1 730 (υ>C = O, $C_{20}$-Carbonyl), 1 615 (υC = C<), 1 055 (υC-O, Acetonid) $cm^{-1}$.

## Beispiel 2

3-Chlor-9α-fluor-11β,16α,17α,21-tetrahydroxy-pregna 1,3,5-trien-20-on-16,17-acetonid

In einem Gemisch aus 750 ml Methanol und 300 ml Benzol werden unter Rühren im Stickstoffstrom 7,42 g (14,55 mMol) 3-Chlor-9α-fluor-11β,16α,17α,21-tetrahydroxy-pregna-1,3,5-trien-20-on-16,17-acetonid-21-acetonid-21-acetat gelöst. Die Lösung wird bei Raumtemperatur 15 Minuten lang gerührt und dann mit der Lösung von 3,0 g (29,96 mMol) Kaliumhydrogencarbonat in 22,5ml destilliertem Wasser versetzt. Das Gemisch wird bei Raumtemperatur weitere drei Stunden lang gerührt, dann wird der pH-Wert (7,5) mit Essigsäure auf 6,5 eingestellt, das Lösungsmittel unter vermindertem Druck bei einer

Temperatur unter 40 °C eingedampft und der feste Rückstand mit 100 ml Eiswasser versetzt. Das feste Produkt wird abfiltriert, mit Eiswasser gewaschen und bei Raumtemperatur, vor Licht geschützt, über Phosphorpentoxyd getrocknet. 6,75 g kristallines Rohprodukt werden erhalten. Dieses wird aus 650 ml Äther umkristallisiert, wobei 5 g (73,5 % der theoretischen Ausbeute) 3-Chlor-9α-fluor-11β,16α,17α,21-tetrahydroxy-pregna-1,3,5-trien-20-on-16,17-acetonid erhalten werden. Das Produkt schmilzt bei bei 229-231 °C.

Elementaranalyse :

Cl gefunden : 7,52 %, 7,97 % (berechnet : 7,83 %).

IR : 3 580, 3 450 (υ-OH), 1 715 (υ>C = O $C_{20}$-Carbonyl), 1 618 (υC = C), 1 055 (υC-O-, Acetonid), 1 382, 1 373 ($\delta_s$-$CH_3$, geminale Methylgruppen im Acetonid) $cm^{-1}$

Beispiel 3

3-Chlor-9α-fluor-11β,16α,17α,21-tetrahydroxy-pregna-1,3,5-trien-20-on-16,17-acetonid-21-monochloracetat

Zu einem Gemisch aus 120 ml Dichlormethan und 21,6 ml Dimethylformamid werden bei 0 °C 6,72 ml (72,8 mMol) Phosphoroxychlorid getropft. Das Gemisch wird bei 0 °C 20 Minuten lang gerührt, dan auf – 10 °C gekühlt und bei dieser Temperatur tropfenweise mit der Lösung von 11,8 g (22,39 mMol) 9α-Fluor-11β,16α,17α,21-tetrahydroxy-pregna-1,4-dien-3,20-dion-16,17-acetonid-21-monochloracetat und 0,24 ml Pyridin in 120 ml Dichlormethan versetzt. Das Gemisch wird 50 Minuten lang auf einer Temperatur von – 5 °C gehalten und dann in die Lösung von 29,16 g Natriumhydrogencarbonat in 800 ml Wasser eingegossen. Nach 30 minütigem Rühren wird der ausgefallene Niederschlag abfiltriert, mit Wasser gewaschen und bei Raumtemperatur im Vakuum getrocknet. 8,45 g (69,3 % der theoretischen Ausbeute) Produkt werden erhalten.

Schmelzpunkt : 266-268 °C.

Elementaranalyse :

Cl gefunden : 12,89 % (berechnet : 13,39 %).

IR : 3 450 (υ-OH), 1 771 (υ>C = O, Chloracetat), 1 729 (υ>C = O, $C_{20}$-Carbonyl), 1 612 (υC = C), 1 378, 1 360 ($\delta_s$-$CH_3$, geminale Methylgruppen im Acetonid), 1 053 (υ C-O-, Acetonid) $cm^{-1}$.

Beispiel 4

3-Chlor-9α-fluor-11β,16α,17α,21-tetrahydroxy-pregna-1,3,5-trien-20-on-16,17-acetonid

In ein Gemisch aus 600 ml Methanol und 240 ml Benzol werden unter Rühren im Stickstoffstrom bei Raumtemperatur 6,35 g (11,66 mMol) 3-Chlor-9α-Fluor-11β,16α,17α,21-tetrahydroxy-pregna-1,3,5-trien-20-on-16,17-acetonid-21-monochloracetat suspendiert. Die Suspension wird 20 Minuten lang gerührt und dann mit der Lösung von 2,4 g (23,97 mMol) Kaliumhydrogencarbonat in 18 ml durch Ionenaustausch entsalztem Wasser versetzt. Das Steroid geht allmählich in Lösung, das Reaktionsgemisch opalisiert jedoch selbst nach zwei Stunden noch. Der pH-Wert des Gemisches wird mit Essigsäure auf 6-6,5 eingestellt, und das Lösungsmittel wird unter vermindertem Druck, bei weniger als 40 °C entfernt. Der feste Rückstand wird mit Wasser vermischt, abfiltriert, mit Wasser gewaschen und dann getrocknet. 5,4 g Rohprodukt werden erhalten. Dieses wird aus Äther umkristallisiert. 4,05 g (74,2 % der theoretischen Ausbeute) 3-Chlor-9α-fluor-11β,16α,17α,21-tetrahydroxy-pregna-1,3,5-trien-20-on-16,17-acetonid werden erhalten.

Schmelzpunkt : 221-222 °C.

Elementaranalyse :

Cl gefunden : 7,64 % (berechnet : 7,83 %)

IR : identisch mit dem des Produktes gemäß Beispiel 2.

Beispiel 5

3-Chlor-6,9α-difluor-11β,16α,17α,21-tetrahydroxy-pregna-1,3,5-trien-20-on-16,17-cetonid-21-monochloracetat

Ein Gemisch aus 10 ml Dichlormethan und 1,53 ml Dimethylformamid wird auf – 10 °C gekühlt und bei dieser Temperatur tropfenweise mit 0,48 ml (5,2 mMol) Phosphoroxychlorid versetzt. Die Lösung wird bei – 10 °C 20 Minuten lang gerührt und dann mit der Lösung von 0,9 g (1,65 mMol) 6α,9α-Difluor-11β,16α,17α,21-tetrahydroxy-pregna-1,4-dien-3,20-dion-16,17-acetonid-21-monochloracetat und 0,02 ml Pyridin in 20 ml Dichlormethan versetzt. Man läßt das Gemisch Raumtemperatur annehmen und rührt dann bei 25-26 °C 5 Stunden lang. Dann wird das Gemisch mit 30 ml Dichlormethan verdünnt und

tropfenweise zu einer Lösung von 2,08 g (20,8 mM) Kaliumhydrogencarbonat in 100 ml Wasser gegeben. Nach 20 minütigem Rühren werden die Phasen voneinander getrennt, die wäßrige Phase wird mit 3 × 30 ml Dichlormethan extrahiert, die organischen Phasen werden vereinigt, mit 2 × 50 ml Wasser gewaschen, über wasserfreiem Natriumsulfat getrocknet und dann filtriert. Das Lösungsmittel wird unter vermindertem Druck bei 30 °C abgedampft und der erhaltene Rückstand mit Äther verrieben, abfiltriert und bei Raumtemperatur, vor Licht geschützt, im Vakuum getrocknet. 0,69 g (74,1 % der theoretischen Ausbeute) Produkt werden erhalten.

Schmelzpunkt : 238-243 °C.

Elementaranalyse :

Cl gefunden : 13,40 %, 13,47 % (berechnet : 12,95 %)

## Beispiel 6

3-Chlor-6,9α-difluor-11β,16α,17α,21-tetrahydroxy-pregna-1,3,5-trien-20-on-16,17-acetonid

In einem Gemisch aus 30 ml Benzol und 80 ml Methanol werden bei Raumtemperatur unter Rühren 0,55 g (0,97 mMol) 3-Chlor-6,9α-difluor-11β,16α,17α,21-tetrahydroxy-pregna-1,3,5-trien-20-on-16,17-acetonid-21-monochloracetat gelöst. Die Lösung wird im Stickstoffstrom 15 Minuten lang gerührt und dann mit der Lösung von 0,2 g (2,0 mMol) Kaliumhydrogencarbonat in 1,5 ml durch Ionenaustausch salzfrei gemachtem Wasser versetzt. Das Reaktionsgemisch beginnt zu opalisieren, wird jedoch nach 15 Minuten wieder klar. Der pH-Wert beträgt 7,5-8. Das Gemisch wird bei 28 °C 35 Minuten lang gerührt, dann mit Essigsäure auf pH 6 eingestellt und bei einer Temperatur von weniger als 40 °C unter vermindertem Druck zur Trockne eingedampft. Der erhaltene feste Rückstand wird mit Wasser verrieben, abfiltriert, mit Wasser gewaschen und im Vakuum, vor Licht geschützt, getrocknet. 0,41 g kristallines Rohprodukt werden erhalten. Dieses wird aus Äther umkristallisiert. 0,37 g (78,2 % der theoretischen Ausbeute) 3-Chlor-6,9α-difluor-11β,16α,17α,21-tetrahydroxy-pregna-1,3,5-trien-20-on-16,17-acetonid werden erhalten.

Schmelzpunkt : 225-229 °C.

IR : 3 510, 3 420 (υ-OH), 1 720 (υ >C = O, $C_{20}$-Carbonyl), 1 678, 1 623 (υC = C), 1 052 (υC-O Acetonid) $cm^{-1}$.

## Beispiel 7

3-Chlor-6-brom-9α-fluor-11β,16α,17α,21-tetrahydroxy-pregna-1,3,5-trien-20-on-16,17-acetonid

Zu dem auf 0 °C gekühlten Gemisch von 10 ml Dichlormethan und 3,6 ml Dimethylformamid werden tropfenweise 1,12 ml (12,2 mMol) Phosphoroxychlorid gegeben. Das Gemisch wird bei 0 °C Minuten lang gerührt, dann auf − 8 °C gekühlt und tropfenweise mit der Lösung von 2,22 g (3,88 mMol) 6-Brom-9α-fluor-11β,16α,17α,21-tetrahydroxy-pregna-1,4-dien-3,20-dion-16,17-acetonid-21-acetat und 0,04 ml Pyridin in 20 ml Dichlormethan versetzt. Das Gemisch wird eine Stunde lang bei einer Temperatur zwischen − 10 °C und 0 °C, dann weitere 4,5 Stunden lang bei Raumtemperatur gerührt und schließlich zum Vollständigmachen der Reaktion bei 2-5 °C 15 Stunden lang stehen gelassen. Danach wird das Gemisch mit 20 ml Dichlormethan verdünnt, mit 20 ml 20 %iger wäßriger Natriumacetatlösung versetzt und bei Raumtemperatur 30 Minuten lang gerührt. Die Phasen werden voneinander getrennt, die wäßrige Phase wird mit 2 × 20 ml Dichlormethan extrahiert, die organischen Phasen werden vereinigt, zuerst mit wäßriger Natriumhydrogencarbonatlösung, dann mit Wasser gewaschen, uber wasserfreiem Natriumsulfat getrocknet und unter vermindertem Druck eingedampft. Als Rückstand werden 2,95 g eines öligen Rohproduktes erhalten. Dieses wird in 30 ml Aceton gelöst, die Lösung wird in Wasser eingetropft, das ausgeschiedene Produkt abfiltriert und getrocknet. 2,0 g 3-Chlor-6-brom-9α-fluor-11β,16α,17α,21-tetrahydroxy-pregna-1,3,5-trien-20-on-16,17-acetonid-21-acetat (87,2 % der theoretischen Ausbeute) werden erhalten. 1,14 g (1,93 mMol) dieses Produktes werden in 40 ml Methanol suspendiert. Zu der Suspension werden unter Rühren bei Raumtemperatur 10,0 ml 67 %ige wäßrige Überchlorsäure tropfenweise zugegeben. Die Suspension wird eine Stunde lang gerührt und dann mit 10 ml Dichlormethan versetzt, wobei sich eine klare Lösung bildet. Diese wird weitere 8 Stunden lang gerührt und dann tropfenweise zu 100 ml 1 %iger wäßriger Natriumhydrogencarbonatlösung gegeben. Das Gemisch wird mit 3 × 50 ml Dichlormethan extrahiert. Die organischen Phasen werden vereinigt, mit 1 %iger wäßriger Natriumhydrogencarbonatlösung und danach mit Wasser neutral gewaschen, über wasserfreiem Natriumsulfat getrocknet und unter vermindertem Druck eingedampft. Das als Rückstand erhaltene kristalline Rohprodukt wird mit dem im Volumverhältnis 1 : 5 bereiteten Gemisch von Äther und Petroläther verrieben, abfiltriert und im Vakuum getrocknet. 1,0 g (94,6 % der theoretischen Ausbeute) 3-Chlor-6-brom-9α-fluor-11β,16α,17α,21-tetrahydroxy-pregna-1,3,5-trien-20-on-16,17-acetonid wird erhalten. Das aus Äther umkristallisierte Produkt schmilzt bei 191-194 °C.

IR : 3 500 (υ$C_{21}$-OH), 3 440 (υ$C_{11}$-OH), 1 715 (υ >C = O $C_{20}$-Carbonyl), 1 638, 1 602, 1 562 (υ C = C), 1 382, 1 375 ($\delta_s$-$CH_3$, germinale Methylgruppen im Acetonid), 1 055 (υ C-O-Acetonid) $cm^{-1}$.

**Patentansprüche** (für die Vertragsstaaten : BE, CH, DE, FR, GB, IT, LI, NL, SE)

1. $\Delta^{1,3,5}$-3-Chlor-11β,16α,17α,21-tetra-(hydroxy)-pregnan-20-on-16,17-acetonidderivate der allgemeinen Formel

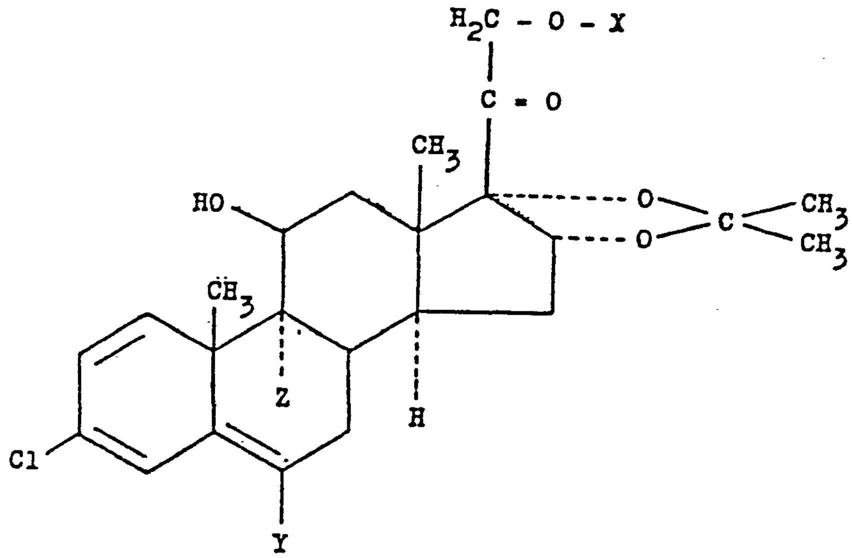

(I)

worin

X für ein Wasserstoffatom oder einen Acetyl- oder Monochloracetylrest steht und

Y und Z jeweils ein Halogen- beziehungsweise Wasserstoffatom bedeuten,

mit der weiteren Maßgabe, daß mindestens 1 von Y und Z von Wasserstoff verschieden ist.

2. $\Delta^{1,3,5}$-3-Chlor-11β,16α,17α,21-tetra-(hydroxy)-pregnan-20-on-16,17-acetonidderivate nach Anspruch 1, dadurch gekennzeichnet, daß das beziehungsweise die Halogenatom(e), für welches beziehungsweise welche Y und/oder Z stehen kann beziehungsweise können, [ein] Fluor-, Brom- und/oder Chloratom(e) ist beziehungsweise sind.

3. 3-(Chlor)-9α-(fluor)-11β,16α,17α,21-tetra-(hydroxy)-pregna-1,3,5-trien-20-on-16,17-acetonid.

4. 3-(Chlor)-6,9α-di-(fluor)-11β,16α,17α,21-tetra-(hydroxy)-pregna-1,3,5-trien-20-on-16,17-acetonid.

5. 3-(Chlor)-6-(brom)-9α-(fluor)-11β,16α,17α,21-tetra-(hydroxy)-pregna-1,3,5-trien-20-on-16,17-acetonid.

6. Verfahren zur Herstellung der Verbindungen nach Anspruch 1 bis 5, dadurch gekennzeichnet, daß man$\Delta^{1,4}$-11β,16α,17α,21-Tetra-(hydroxy)-pregnan-3,20-dion-16,17-acetonid-21-acetat- beziehungsweise $\Delta^{1,4}$-11β,16α,17α,21-Tetra-(hydroxy)-pregnan-3,20-dion-16,17-acetonid-21-monochloracetatderivate der allgemeinen Formel

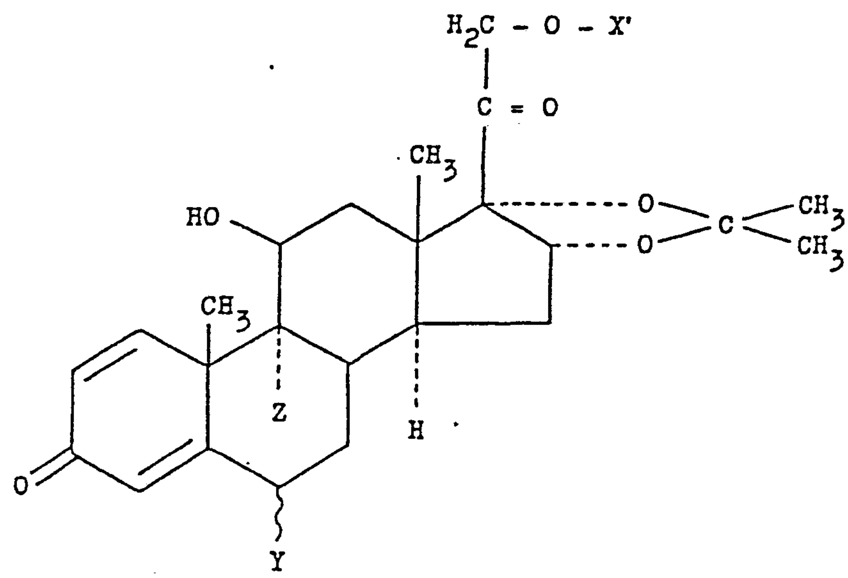

(II)

worin

X' für einen Acetyl- oder Monochloracetylrest steht und

Y und Z wie im Anspruch 1 oder 2 festgelegt sind,

in aprotonischen Lösungsmitteln in Gegenwart von 1 oder mehr tertiären Base(n) mit Chlormethyleniminiumsalzen der allgemeinen Formel

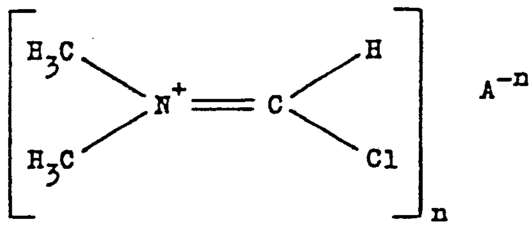

(III)

worin

$A^-$ für ein salzbildendes Anion steht und

n 1, 2 oder 3 ist,

zu $\Delta^{1,3,5}$-3-Chlor-11β,16α,17α,21-tetra-(hydroxy)-pregnan-20-on-16,17-acetonidderivaten der allgemeinen Formel I, bei welchen X für einen Acetyl- oder Monochloracetylrest steht, umsetzt und gegebenenfalls die letzteren in an sich bekannter Weise zu den entsprechenden $\Delta^{1,3,5}$-3-Chlor-11,16α,17α,21-tetra-(hydroxy)-pregnan-20-on-16,17-acetonidderivaten der allgemeinen Formel I, bei welchen X für Wasserstoff steht, hydrolysiert.

7. Verfahren nach Anspruch 6, dadurch gekennzeichnet, daß man als Chlormethyleniminiumsalze der allgemeinen Formel III solche, bei welchen $A^-$ für ein Dichlorphosphatanion

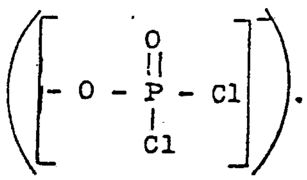

steht, verwendet.

8. Verfahren nach Anspruch 6 oder 7, dadurch gekennzeichnet, daß man als aprotonische[s] Lösungsmittel 1 oder mehr niedere[n] Chlorkohlenwasserstoff(e) und/oder Dimethylformamid verwendet.

9. Verfahren nach Anspruch 6 bis 8, dadurch gekennzeichnet, daß man als tertiäre Base Pyridin und/oder 1 oder mehr Monoalkyl- und/oder Polyalkylderivat(e) desselben verwendet.

10. Arzneimittel, gekennzeichnet durch einen Gehalt an 1 oder mehr Verbindung(en) nach Anspruch 1 bis 5 als Wirkstoff(en), zweckmäßig zusammen mit 1 oder mehr üblichen pharmazeutischen Konfektionierungsmittel(n).

**Patentansprüche** (für den Vertragsstaat AT)

1. Verfahren zur Herstellung von $\Delta^{1,3,5}$-3-Chlor-11β,16α,17α,21-tetra-(hydroxy)-pregnan-20-on-16,17-acetonidderivaten der allgemeinen Formel

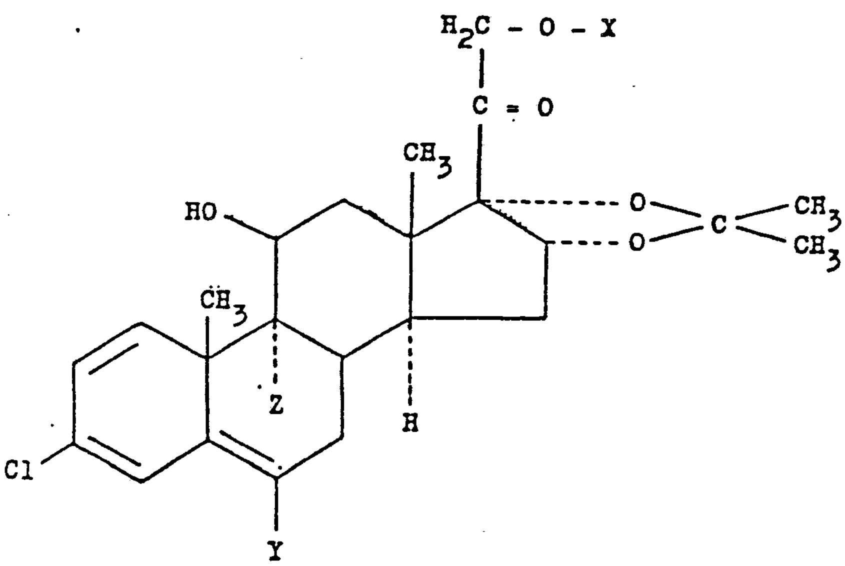

(I)

worin

X für ein Wasserstoffatom oder einen Acetyl- oder Monochloracetylrest steht und

Y und Z jeweils ein Halogen- beziehungsweise Wasserstoffatom bedeuten,

mit der weiteren Maßgabe, daß mindestens 1 von Y und Z von Wasserstoff verschieden ist, dadurch gekennzeichnet, daß man $\Delta^{1,4}$-11β,16α,17α,21-Tetra-(hydroxy)-pregnan-3,20-dion-16,17-acetonid-21-acetat- beziehungsweise $\Delta^{1,4}$-11β,16α,17α,21-Tetra-(hydroxy)-pregnan-3,20-dion-16,17-acetonid-21-monochloracetatderivate der allgemeinen Formel

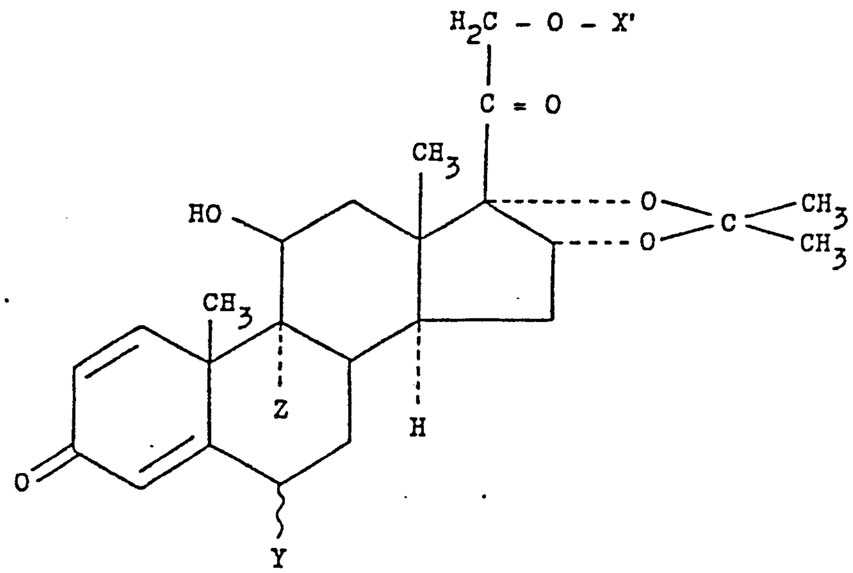

(II)

worin

X' für einen Acetyl- oder Monochloracetylrest steht und

Y und Z wie im Anspruch 1 oder 2 festgelegt sind,

in aprotonischen Lösungsmitteln in Gegenwart von 1 oder mehr tertiären Base(n) mit Chlormethyleniminiumsalzen der allgemeinen Formel

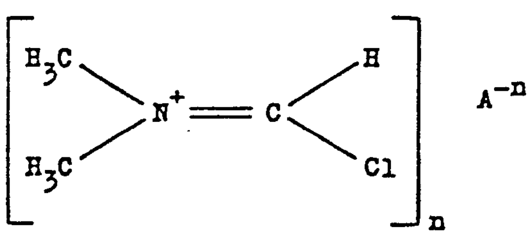

(III)

worin

$A^-$ für ein salzbildendes Anion steht und

n 1, 2 oder 3 ist,

zu $\Delta^{1,3,5}$-3-Chlor-11β,16α,17α,21-tetra-(hydroxy)-pregnan-20-on-16,17-acetonidderivaten der allgemeinen Formel I, bei welchen X für einen Acetyl- oder Monochloracetylrest steht, umsetzt und gegebenenfalls die letzteren in an sich bekannter Weise zu den entsprechenden $\Delta^{1,3,5}$-3-Chlor-11β,16α,17α,21-tetra-(hydroxy)-pregnan-20-on-16,17-acetonidderivaten der allgemeinen Formel I, bei welchen X für Wasserstoff steht, hydrolysiert.

2. Verfahren nach Anspruch 1 dadurch gekennzeichnet, daß man als Chlormethyleniminiumsalze der allgemeinen Formel III solche, bei welchen $A^-$ für ein Dichlorphosphatanion

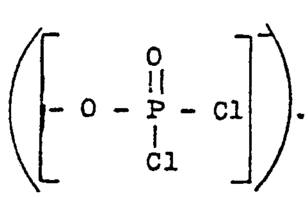

steht, verwendet.

3. Verfahren nach Anspruch 1 oder 2, dadurch gekennzeichnet, daß man als aprotonische[s] Lösungsmittel 1 oder mehr niedere[n] Chlorkohlenwasserstoff(e) und/oder Dimethylformamid verwendet.

4. Verfahren nach Anspruch 1 bis 3 dadurch gekennzeichnet, daß man als tertiäre Base Pyridin und/oder 1 oder mehr Monoalkyl- und/oder Polyalkylderivate(e) desselben verwendet.

5. Verfahren nach Anspruch 1 bis 4, dadurch gekennzeichnet, daß man $\Delta^{1,3,5}$-3-Chlor-11β,16α,17α,21-tetra-(hydroxy)-pregnan-20-on-16,17-acetonidderivate, in welchen das beziehungsweise die Halogenatom(e), für welches beziehungsweise welche Y und/oder Z stehen kann beziehungsweise können, [ein] Fluor-, Brom- und/oder Chloratom(e) ist beziehungsweise sind, herstellt.

6. Verfahren nach Anspruch 1 bis 5, dadurch gekennzeichnet, daß man 3-(Chlor)-9α-(fluor)-11β,16α,17α,21-tetra-(hydroxy)-pregna-1,3,5-trien-20-on-16,17-acetonid herstellt.

7. Verfahren nach Anspruch 1 bis 5, dadurch gekennzeichnet, daß man 3-(Chlor)-6,9α-di-(fluor)-11β-16α,17α,21-tetra-(hydroxy)-pregna-1,3,5-trien-20-on-16,17-acetonid herstellt.

8. Verfahren nach Anspruch 1 bis 5, dadurch gekennzeichnet, daß man 3-(Chlor)-6-(brom)-9α-(fluor)-11β,16α,17α,21-tetra-(hydroxy)-pregna-1,3,5-trien-20-on-16,17-acetonid herstellt.

**Claims** (for the Contracting States : BE, CH, DE, FR, GB, IT, LI, NL, SE)

1. $\Delta^{1,3,5}$-3-chloro-11ß,16α,17α,21-tetra-(hydroxy)-pregnane-20-one-16,17-acetonide derivatives having the general formula

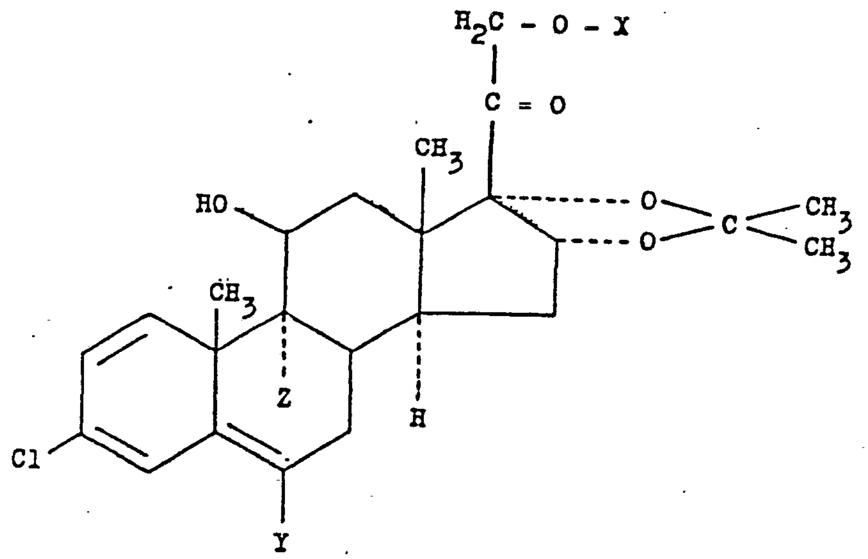

(I)

wherein

X represents a hydrogen atom or an acetyl or monochloroacetyl radical and

Y and Z each mean a halogen or hydrogen atom, respectively,

with the further provision that at least 1 of Y and Z is different from hydrogen.

2. $\Delta^{1,3,5}$-3-chloro-11β,16α,17α,21-tetra-(hydroxy)-pregnane-20-one-16,17-acetonide derivatives according to claim 1, characterized in that the halogen atom(s), which may be represented by Y and/or Z is ore are, respectively, [a] fluorine, bromine and/or chlorine atom(s).

3. 3-(chloro)-9α-(fluoro)-11β,16α,17α,21-tetra-(hydroxy)-pregna-1,3,5-triene-20-one-16,17-acetonide.

4. 3-(chloro)-6,9α-di-(fluoro)-11β,16α,17α,21-tetra-(hydroxy)-pregna-1,3,5-triene-20-one-16,17-acetonide.

5. 3-(chloro)-6-(bromo)-9α-(fluoro)-11β,16α,17α,21-tetra-(hydroxy)-pregna-1,3,5-triene-20-one-16,17-acetonide.

6. A process for preparing the compounds according to claims 1 to 5, characterized in that onereacts $\Delta^{1,4}$-11β,16α,17α,21-tetra-(hydroxy)-pregnane-3,20-dione-16,17-acetonide-21-acetate or $\Delta^{1,4}$-11β,16α,17α,21-tetra-(hydroxy)-pregnane-3,20-dione-16,17-acetonide-21-monochloroacetate derivatives, respectively, having the general formula

(See Figure page 18)

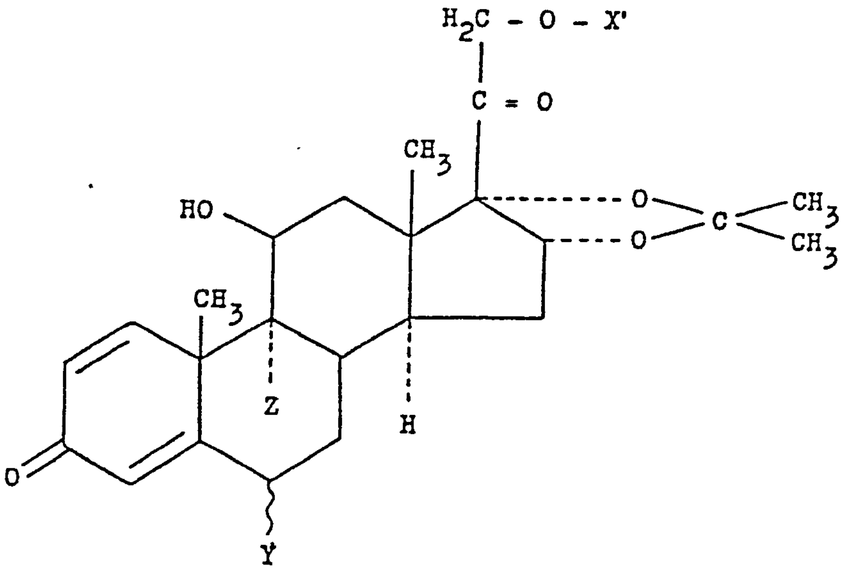

(II)

wherein

X' represents an acetyl or monochloroacetyl radical and

Y and Z are defined as in claim 1 or 2

in aprotic solvents in the presence of 1 or more tertiary base(s) with chloromethyleneiminium salts having the general formula

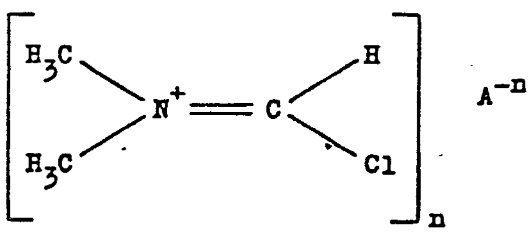

(III)

wherein

$A^-$ represents a salt-forming anion and

n is 1, 2 or 3

to yield $\Delta^{1,3,5}$-3-chloro-11β,16α,17α,21-tetra-(hydroxy)-pregnane-20-one-16,17-acetonide derivatives having the general formula I, in which X represents an acetyl or monochloroacetyl radical and optionally on hydrolyzes the latter in a manner known per se to yield the corresponding $\Delta^{1,3,5}$-3-chloro-11β,16α,17α,21-tetra-(hydroxy)-pregnane-20-one-16,17-acetonide derivatives having the general formula I in which X represents hydrogen.

7. A process according to claim 6, characterized in that one uses as chloromethyleneiminium salts of the general formula III such in which $A^-$ represents a dichlorophosphate anion

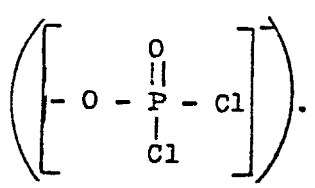

8. A process according to claim 6 or 7, characterized in that one uses as [an] aprotic solvent(s) 1 or more lower chlorinated hydrocarbon(s) and/or dimethylformamide.

9. A process according to claims 6 to 8, characterized in that one uses as a tertiary base pyridine and/or 1 or more monoalkyl and/or polyalkyl derivative(s) of it.

10. Medicaments, characterized by a content of 1 or more compound(s) according to claims 1 to 5 as [an] active principle(s), usefully together with 1 or more usual pharmaceutical processing agent(s).

**Claims** (for the Contracting State AT)

1. A process for preparing $\Delta^{1,3,5}$-3-chloro-11β-16α,17α,21-tetra-(hydroxy)-pregnane-20-one-16,17-acetonide derivatives having the general formula

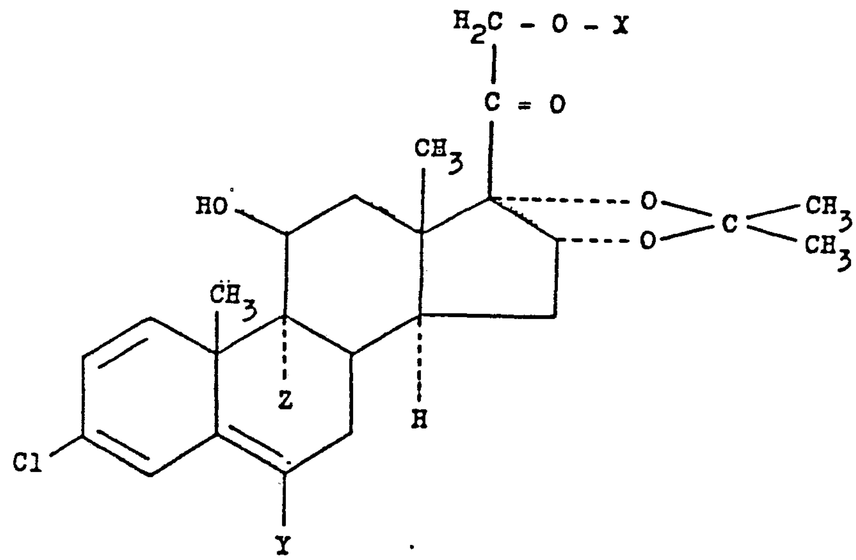

(I)

wherein

X represents a hydrogen atom or an acetyl or monochloroacetyl radical and

Y and Z each mean a halogen or hydrogen atom, respectively,

with the further provision that at least 1 of Y and Z is different from hydrogen, characterized in that one reacts $\Delta^{1,4}$-11β,16α,17α,21-tetra-(hydroxy)-pregnane-3,20-dione-16,17-acetonide-21-acetate or $\Delta^{1,4}$-11β,16α,17α,21-tetra-(hydroxy)-pregnane-3,20-dione-16,17-acetonide-21-monochloroacetate derivatives, respectively, having the general formula

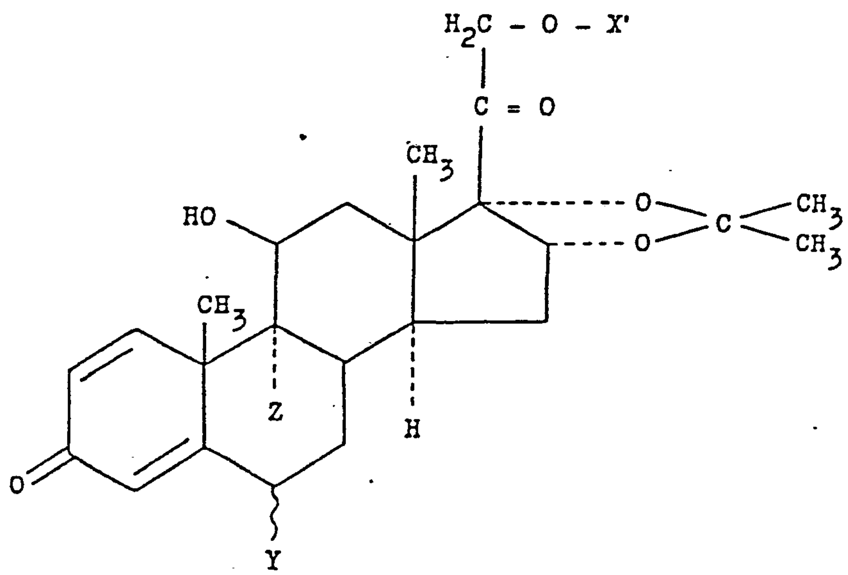

(II)

wherein

X' represents an acetyl or monochloroacetyl radical and

Y and Z are defined as in claim 1 or 2

in aprotic solvents in the presence of 1 or more tertiary base(s) with chloromethyleneiminium salts having the general formula

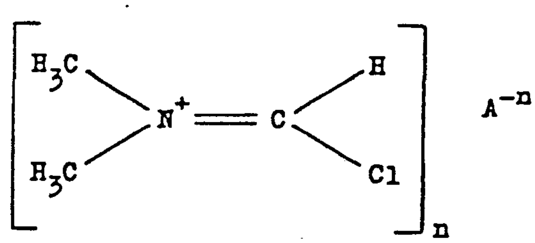

(III)

wherein

$A^-$ represents a salt-forming anion and

n is 1, 2 or 3

to yield $\Delta^{1,3,5}$-3-chloro-11β,16α,17α,21-tetra-(hydroxy)-pregnane-20-one-16,17-acetonide derivatives having the general formula I, in which X represents an acetyl or monochloroacetyl radical and optionally one hydrolyzes the latter in a manner known per se to yield the corresponding $\Delta^{1,3,5}$-3-chloro-11β,16α,17α,21-tetra-(hydroxy)-pregnane-20-one-16,17-acetonide derivatives having the general formula I in which X represents hydrogen.

2. A process according to claim 1, characterized in that one uses as chloromethyleneiminium salts of the general formula III such in which $A^-$ represents a dichlorophosphate anion

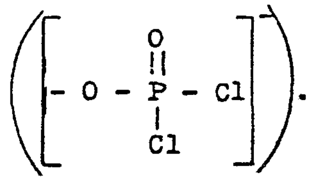.

3. A process according to claim 1 or 2, characterized in that one uses as [an] aprotic solvent(s) 1 or more lower chlorinated hydrocarbon(s) and/or dimethylformamide.

4. A process according to claims 1 to 3, characterized in that one uses as a tertiary base pyridine and/or 1 or more monoalkyl and/or polyalkyl derivative(s) of it.

5. A process according to claims 1 to 4, characterized in that one prepares $\Delta^{1,3,5}$-3-chloro-11β,16α,17α,21-tetra-(hydroxy)-pregnane-20-one-16,17-acetonide derivatives in which halogen atom(s), which may be represented by Y and/or Z is or are, respectively, [a] fluorine, bromine and/or chlorine atom(s).

6. A process according to claims 1 to 5, characterized in that one prepares 3-(chloro)-9α-(fluoro)-11β,16α,17α,21-tetra-(hydroxy)-pregna-1;3,5-triene-20-one-16,17-acetonide.

7. A process according to claims 1 to 5, characterized in that one prepares 3-chloro)-6,9α-di-(fluoro)-11β,16α,17α,21-tetra-(hydroxy)-pregna-1,3,5-triene-20-one-16,17-acetonide.

8. A process according to claims 1 to 5, characterized in that one prepares 3-(chloro)-6-(bromo)-9α-(fluoro)-11β,16α,17α,21-tetra-(hydroxy)-pregna-1,3,5-triene-20-one-16,17-acetonide.

**Revendications** (pour les Etats contractants : BE, CH, DE, FR, GB, IT, LI, NL, SE)

1. Dérivés $\Delta^{1,3,5}$-3-chloro-11β,16α,17α,21-tétrahydroxypregnane-20-one-16,17-acétonides de formule générale

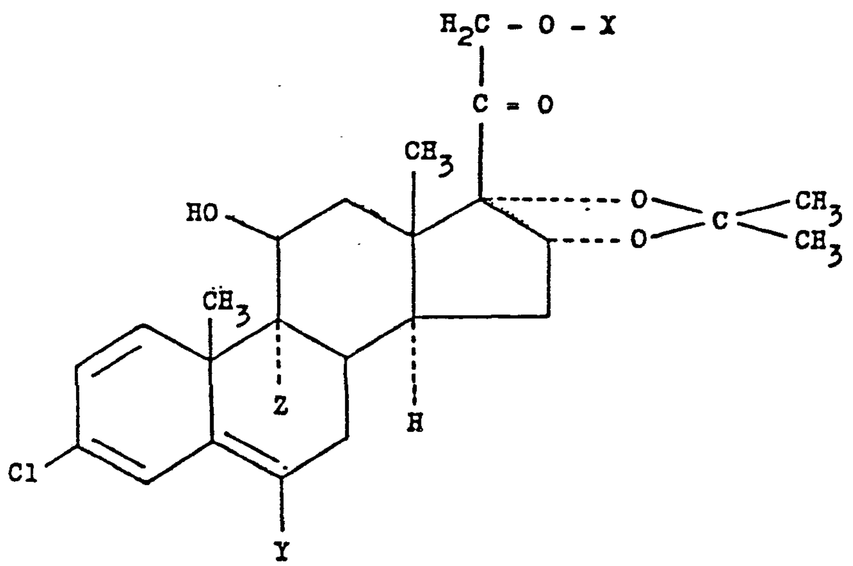

(I)

dans laquelle

X représente un atome d'hydrogène ou un radical acétyle ou monochloracétyle et

Y et Z représentent chacun un atome d'halogène ou d'hydrogène, avec cette autre condition qu'au moins un de Y et Z est différent de l'hydrogène.

2. Dérivés $\Delta^{1,3,5}$-3-chloro-11β,16α,17α,21-tétrahydroxypregnane-20-one-16,17-acétonides selon la revendication 1, caractérisés par le fait que le ou les atomes d'halogène que peuvent représenter Y et/ou Z sont un ou des atomes de fluor, de brome et/ou de chlore.

3. 3-Chloro-9α-fluoro-11β,16α,17α,21-tétrahydroxypregna-1,3,5-triène-20-one-16,17-acétonide.

4. 3-Chloro-6,9α-difluoro-11β,16α,17α,21-tétrahydroxypregna-1,3,5-triène-20-one-16,17-acétonide.

5. 3-chloro-6-bromo-9α-fluoro-11β,16α,17α,21-tétrahydroxypregna-1,3,5-triène-20-one-16,17-acétonide.

6. Procédé de préparation des composés selon les revendications 1 à 5, caractérisé par le fait que l'on fait réagir le 21-acétate de $\Delta^{1,4}$-11β,16α,17α,21-tétrahydroxypregnane-3,20-dione-16,17-acétonide ou des dérivés 21-monochloracétates de $\Delta^{1,4}$-11β,16α,17α,21-tétrahydroxypregnane-3,20-dione-16,17-acétonides de formule générale

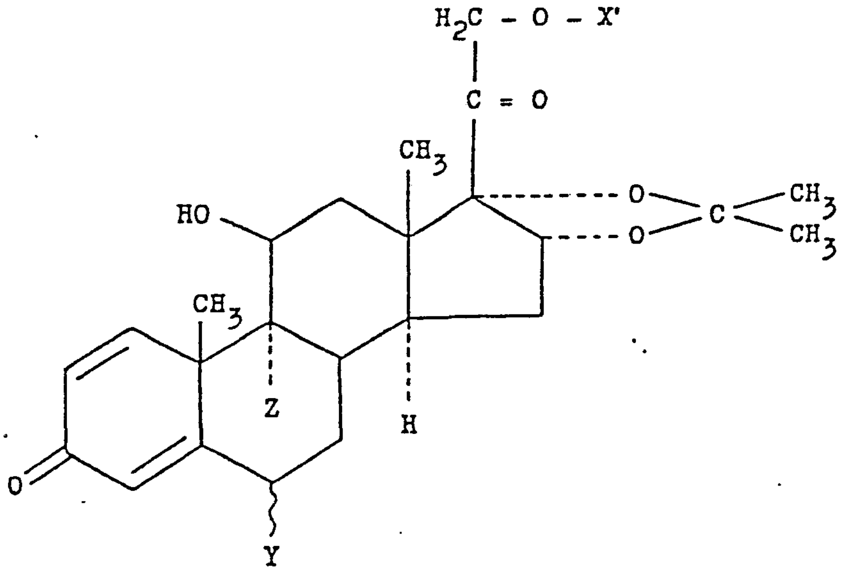

(II)

dans laquelle

X représente un radical acétyle ou monochloracétyle et

Y et Z sont déterminés comme dans l'une des revendications 1 et 2,

dans des solvants aprotoniques, en présence d'une ou plusieurs basés tertiaires, sur des sels de chlorométhylène-iminium de formule générale

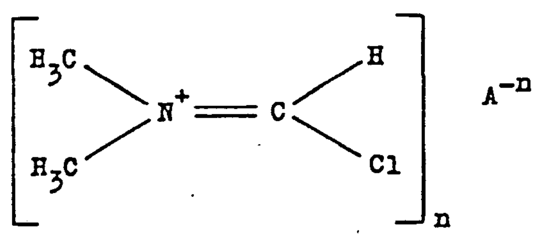

(III)

dans laquelle

$A^-$ représente un anion salifiable et

n vaut 1, 2 ou 3,

pour former des dérivés $\Delta^{1,3,5}$-3-chloro-11β,16α,17α,21-tétrahydroxypregnane-20-one-16,17-acétonides de formule générale I dans lesquels X représente un radical acétyle ou monochloracétyle et qu'éventuellement on hydrolyse ces derniers de manière en elle-même connue pour former les dérivés $\Delta^{1,3,5}$-3-chloro-11β,16α,17α,21-tétrahydroxypregnane-20-one-16,17-actonides correspondants de formule générale I dans lesquels X représente l'hydrogène.

7. Procédé selon la revendication 6, caractérisé par le fait que comme sels de chlorométhylène-iminium de formule générale III, on utilise ceux dans lesquels $A^-$ représente un anion dichlorophosphate

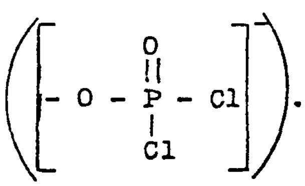

8. Procédé selon l'une des revendications 6 et 7, caractérisé par le fait que comme solvant(s) aprotonique(s), on utilise un ou plusieurs chlorohydrocarbures inférieurs et/ou la diméthylformamide.

9. Procédé selon les revendications 6 à 8, caractérisé par le fait que comme base tertiaire, on utilise la pyridine et/ou un ou plusieurs dérivés monoalkylés et/ou polyalkylés de celle-ci.

10. Médicament caractérisé par une teneur en un ou plusieurs composés selon les revendications 1 à 5, comme substance(s) active(s), avantageusement en même temps qu'un ou plusieurs agents de conditionnement pharmaceutiques usuels.

**Revendications** (pour l'Etat contractant AT)

1. Procédé de préparation des dérivés $\Delta^{1,3,5}$-3-chloro-11β,16α,17α,21-tétrahydroxypregnane-20-one-16,17-acétonides de formule générale

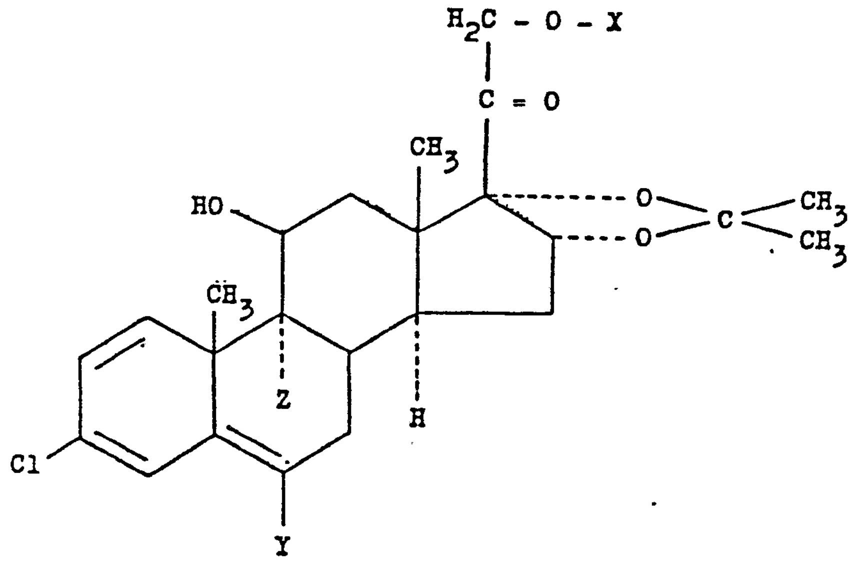

(I)

dans laquelle

X représente un atome d'hydrogène ou un radical acétyle ou monochloracétyle et

Y et Z représentent chacun un atome d'halogène ou d'hydrogène, avec cette autre condition qu'au moins un de Y et Z est différent de l'hydrogène, caractérisé par le fait que l'on fait réagir le 21-acétate de $\Delta^{1,4}$-11β,16α,17α,21-tétrahydroxypregnane-3,20-dione-16,17-acétonide ou des dérivés 21-monochloracétates de $\Delta^{1,4}$-11β,16α,17α,21-tétrahydroxypregnane-3,20-dione-16,17-acétonides de formule générale :

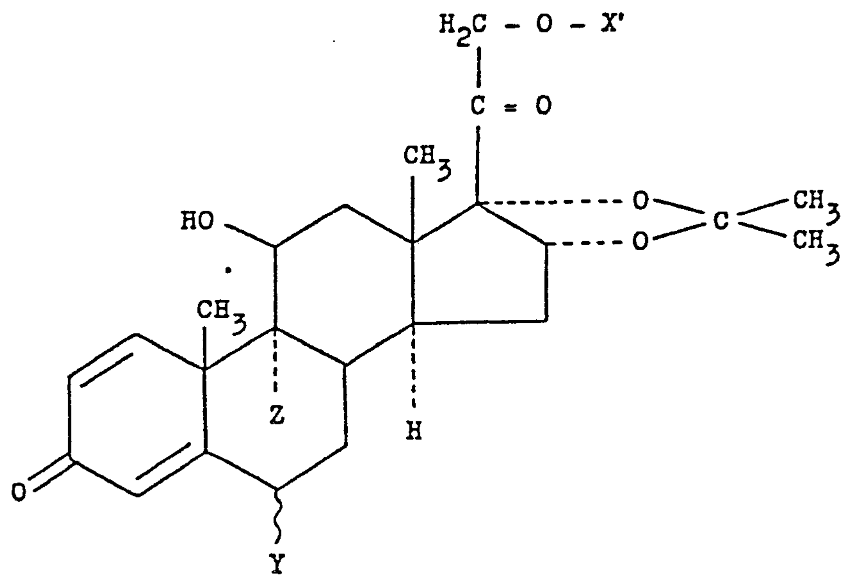

(II)

dans laquelle

X représente un radical acétyle ou monochloracétyle et Y et Z sont déterminés comme dans l'une des revendications 1 et 2,

dans des solvants aprotoniques, en présence d'une ou plusieurs bases tertiaires, sur des sels de chlorométhylène-iminium de formule générale

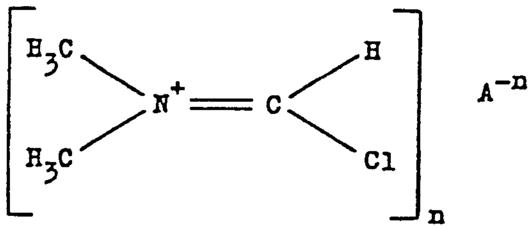

(III)

dans laquelle

A⁻ représente un anion salifiable et

n vaut 1, 2 ou 3,

pour former des dérivés $\Delta^{1,3,5}$-3-chloro-11β,16α,17α,21-tétrahydroxypregnane-20-one-16,17-acétonides de formule générale I dans lesquels X représente un radical acétyle ou monochloracétyle et qu'éventuellement on hydrolyse ces derniers de manière en elle-même connue pour former les dérivés $\Delta^{1,3,5}$-3-chloro-11β,16α,17α,21-tétrahydroxypregnane-20-one-16,17-acétonides correspondants de formule générale I dans lesquels X représente l'hydrogène.

2. Procédé selon la revendication 1, carctérisé par le fait que comme sels de chlorométhylène-iminium de formule générale III, on utilise ceux dans lesquels A⁻ représente un anion dichlorophosphate

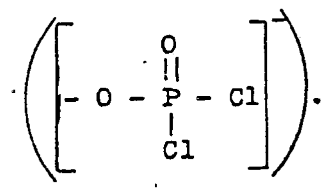

3. Procédé selon l'une des revendications 1 et 2, caractérisé par le fait que comme solvant(s) aprotonique(s), on utilise un ou plusieurs chlorohydrocarbures inférieurs et/ou la diméthylformamide.

4. Procédé selon les revendications 1 à 3, caractérisé par le fait que comme base tertiaire, on utilise la pyridine et/ou un ou plusieurs dérivés monoalkylés et/ou polyalkylés de celle-ci.

5. Procédé selon les revendications 1 à 4 caractérisé par le fait que l'on prépare dérivés $\Delta^{1,3,5}$-3-chloro-11β,16α,17α,21-tétrahydroxypregnane-20-one-16,17-acétonides dans lesquelles le ou les atomes d'halogène que peuvent représenter Y et/ou Z sont un ou des atomes de fluor, de brome et/ou de chlore.

6. Procédé selon les revendications 1 à 5 caractérisé par le fait que l'on prépare 3-chloro-9α-fluoro-11β,16α,17α,21-tétrahydroxypregna-1,3,5-triène-20-one-16,17-acétonide.

7. Procédé selon les revendications 1 à 5 caractérisé par le fait que l'on prépare 3-chloro-6,9α-difluoro-11β,16α,17α,21-tétrahydroxypregna-1,3,5-triène-20-one-16,17-acétonide.

8. Procédé selon les revendications 1 à 5 caractérisé par le fait que l'on prépare 3-chloro-6-bromo-9α-fluoro-11β,16α,17α,21-tétrahydroxypregna-1,3,5-triène-20-one-16,17-acétonide.